(19) Europäisches Patentamt
European Patent Office
Office européen des brevets

(11) EP 3 253 374 B1

(12) **EUROPEAN PATENT SPECIFICATION**

(45) Date of publication and mention of the grant of the patent:
**28.11.2018 Bulletin 2018/48**

(21) Application number: **16706540.8**

(22) Date of filing: **01.02.2016**

(51) Int Cl.:
***A61K 9/14*** *(2006.01)* ***A61K 31/407*** *(2006.01)*

(86) International application number:
**PCT/EP2016/052017**

(87) International publication number:
**WO 2016/124513 (11.08.2016 Gazette 2016/32)**

(54) **COMPOSITION COMPRISING CEBRANOPADOL IN A DISSOLVED FORM**

ZUSAMMENSETZUNG MIT CEBRANOPADOL IN GELÖSTER FORM

COMPOSITION COMPRENANT DU CEBRANOPADOL SOUS FORME DISSOUTE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **02.02.2015 EP 15153405**

(43) Date of publication of application:
**13.12.2017 Bulletin 2017/50**

(73) Proprietor: **ratiopharm GmbH**
**89079 Ulm (DE)**

(72) Inventors:
- **ALBRECHT, Wolfgang**
  **89075 Ulm (DE)**
- **GEIER, Jens**
  **72534 Hayingen (DE)**
- **LEHMANN, Frank**
  **89233 Neu-Ulm (DE)**

(74) Representative: **Ter Meer Steinmeister & Partner**
**Patentanwälte mbB**
**Nymphenburger Straße 4**
**80335 München (DE)**

(56) References cited:
**EP-A1- 2 251 038**
**WO-A2-2012/016703**
**WO-A2-2013/030535**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 253 374 B1

## Description

Background of the invention

The present invention relates to a composition comprising cebranopadol in dissolved form and oral dosage forms comprising said composition. The invention further relates to a process for producing the composition comprising cebranopadol in a dissolved form and to the corresponding process of producing an oral dosage form containing the composition of the invention.

Cebranopadol is an analgesic which is reported to have a unique mechanism of action. Cebranopadol is supposed to bind to and activate the nociception-receptor (also known as orphanin FQ-receptor) as well as the classic opioid receptors, for example $\mu$-opioid receptor. By this mechanism of action cebranopadol is suggested to be a potent analgesic for medium to strong chronic pain including neuropathic pain, as such in patients with tumor(s).

In the context of this invention, the IUPAC name of "cebranopadol" is (1r,4r)-6'-fluoro-*N*,*N*-dimethyl-4-phenyl-4',9'-dihydro-3'*H*-spiro[cyclohexane-1,1'-pyrano[3,4-*b*]indol]-4-amine, which is represented by the following Formula (I).

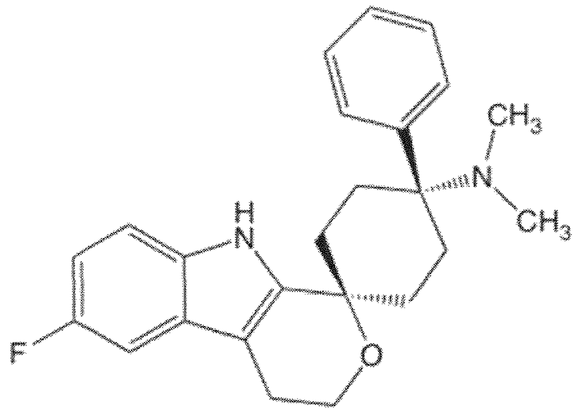

Formula (I)

Synthesis pathways for cebranopadol and addition salts thereof and the use as an analgesic have been described for example in EP 1 560 835 B1.

Further, WO 2013/007361 A1 discloses crystalline polymorphic forms of cebranopadol and cebranopadol solvates which can be distinguished by X-ray diffraction. It is further reported that the crystalline polymorphs might convert into each other, for example in the presence of solvents or when heat or a mechanical energy is applied. However, such a behaviour (occurrence of morphological changes) can be unfavourable for example for dosage forms such as tablets, since it may cause solid state changes in the dosage form often resulting in different dissolution and pharmacokinetic properties. Such changes hence may require a strict temperature control of the dosage forms, especially in summer and/or in climate zones III and IV. Additionally, such solid state changes may lead to regulatory and commercial disadvantages.

Further, cebranopadol is reported to be poorly soluble and to have to be applied in very small amounts (< 1 mg). Pharmaceutical compositions comprising said active pharmaceutically ingredients with said properties tend to have problems with the solubility/bioavailability and especially with the content uniformity of the active pharmaceutical ingredient. Since the active pharmaceutical ingredient is administered in very low doses, even slightly inhomogeneous pharmaceutical compositions, for example due to little degradation, can lower the beneficial effects significantly or may even be the basis for undesirable side effects.

WO 2012/016703 A2 describes a pharmaceutical dosage form comprising cebranopadol. Said dosage from comprises a liquid core comprising cebranopadol in a dispersed or dissolved form surrounded by an encapsulating material. Such a system might be regarded as a self-emulsifying drug delivery system (SEDDS). However, the production of such SEDDS is complex and thus time and cost-consuming. Additionally, the amounts of the surfactants to produce the liquid core of the dosage might cause undesirable side effects to the gastrointestinal tract, such as diarrhea.

Hence, it was an object of the present invention to overcome the above drawbacks of the above-mentioned formulation.

In particular, it was an object of the present invention to provide cebranopadol in a form in which does not require a specific temperature control during storage or when used in climate zones III and IV.

Further, a form of cebranopadol should be provided that shows advantageous dissolution and pharmacokinetic

properties, in particular when used after storage or in in climate zones III and IV.

Further, a form of cebranopadol should be provided that shows improved properties with regard to processability.

Additionally, it was an object to provide a pharmaceutical composition, wherein the composition does not contain an encapsulated liquid core as described in WO 2012/016703 and/or polymorphs as described in WO 2013/007361. Nevertheless, the composition or dosage form of the present invention should provide in-vitro and in-vivo properties being similar to the formulation as described in 2012/016703 and/or to the polymorphs as described in WO 2013/007361.

All the above-mentioned objectives should preferably be solved for a dosage form designed for immediate release ("IR") and for modified release ("MR"). In particular a rapid pain relief but a long lasting therapeutic effect is desired.

Summary of the Invention

According to the present invention, the above objectives can be achieved by a pharmaceutical composition comprising dissolved cebranopadol, organic solvent with a high boiling point and carrier. In the composition of the present invention cebranopadol is present in a dissolved form in an organic solvent with a high boiling point and may adhere to said carrier or is preferably adsorbed on said carrier in dissolved form. The composition can advantageously be processed into oral dosage forms.

Thus, the subject of the invention is a pharmaceutical composition, preferably a pharmaceutical composition having a solid appearance, comprising

(a) dissolved cebranopadol,
(b) organic solvent with a boiling point of 110 to 350°C at 1013 mbar, and
(c) solid carrier.

Atmospheric pressure, 1013 mbar and 760 mm Hg are equivalent values and refer to standard pressure.

A further subject of the present invention is an oral dosage form comprising the composition of the invention and optionally further pharmaceutical excipient(s).

Another subject of the present invention is a method of producing the composition according to the invention comprising the steps of

i) dissolving cebranopadol (a) in organic solvent (b) with a boiling point of 110 to 350°C, wherein the boiling point is measured at 1013 mbar
ii) mixing solid carrier (c) and the solution of step i)
iii) optionally milling and/or sieving the mixture of step ii).

Further, the subject of the present invention relates to a process for producing an oral dosage form comprising the steps of

i) dissolving cebranopadol (a) in organic solvent (b) with a boiling point of 110 to 350°C, wherein the boiling point is measured at 1013 mbar
ii) mixing solid carrier (c) and the solution of step i)
iii) optionally milling and/or sieving the mixture of step ii)
iv) optionally adding further excipient(s) to the mixture of step ii) or step iii),
v) optionally granulating the mixture of step ii), step iii) or step iv)
vi) processing the mixture of step ii), step iii) or step iv) or the granulates of step v) into an oral dosage form.

The above-illustrated subjects of the present invention are alternative solutions to the above-outlined problems.

Detailed Description of the Invention

The present invention concerns a pharmaceutical composition which is a mixture of a solid state carrier and cebranopadol in dissolved state. Preferably, the physical appearance of the entire composition is solid at 25°C and 1013 mbar. The same applies to the dosage form of the present invention.

In the context of this invention, cebranopadol may refer to cebranopadol in the form of the free base. Further, cebranopadol may refer to pharmaceutically acceptable salts, hydrates, solvates, polymorphs, stereoisomers, like enantiomers, and mixtures thereof. For example, the invention also refers to enantiomers of pharmaceutical acceptable salts of cebranopadol, to solvates of salts or hydrates of polymorphs or the like.

In a preferred embodiment of the invention cebranopadol refers to as cebranopadol in form of the free base.

In an alternative preferred embodiment cebranopadol refers to pharmaceutically acceptable salts thereof, preferably pharmaceutically acceptable acid addition salts. The acids which can be used to prepare the pharmaceutically acceptable acid addition salts are preferably those which form non-toxic acid addition salts. Non-toxic acids for forming acid salts are for example hydrochloric acid, hydrobromic acid, hydroiodic acid, nitric acid, sulfuric acid, phosphoric acid, formic acid, acetic acid, oxalic acid, lactic acid, citric acid, tartaric acid, succinic acid, maleic acid, fumaric acid, mandelic acid, gluconic acid, saccharic acid, glutamic acid, benzoic acid, 2,4,6-trimethylbenzoic acid, acetylsalicylic acid, methanesulfonic acid, ethanesulfonic acid, benzenesulfonic acid, p-toluenesulfonic acid and pamoic acid [1,1'-methylene-bis-(2-hydroxy-3-naphthoic acid, hippuric acid sand nicotinic acid.

In case the weight of cebranopadol is mentioned in the present invention, this refers to cebranopadol in its free form.

In a particularly preferred embodiment the composition of the present invention as well as the oral dosage form of the present invention comprise cebranopadol as the sole pharmaceutical active agent.

In an alternative embodiment the composition of the present invention as well as the oral dosage form of the present invention can comprise cebranopadol in combination with further pharmaceutical active agent(s).

In the present invention cebranopadol is present in a dissolved form.

The term "dissolved cebranopadol" can be used in the context of this invention to designate the above compound in which the components (atoms, ions or molecules) do not exhibit a periodic arrangement over a great range (= long-range order), such as usually known from crystalline substances. Consequently, the present cebranopadol for example does not show any clear interferences determined by means of X-ray diffraction. The present cebranopadol components (atoms, ions or molecules, preferably atoms) are each preferably surrounded by a solvate shell. This solvate shell can be composed of several layers of solvent molecules wherein the molecules of the various layers of the solvate shell interact with the core molecule the more, the closer they are to said core molecule. Solvated molecules can preferably be regarded as a flexible entity whose solvate shell is in interaction with solvent molecules.

In a preferred embodiment dissolved cebranopadol can be regarded as non-solid cebranopadol or in other words cebranopadol in a non-solid form.

The organic solvent (b) has a boiling point of 110°C to 350°C, wherein the boiling point is measured at 1013 mbar. However, this does not imply that the cebranopadol has to be dissolved at 1013 mbar in the organic solvent (b). Further preferred, the organic solvent can have a boiling point of at least 120°C, 130°C, 140°C, 150°C, 160°C, 170°C, 180°C or 190°C. Further preferred, the organic solvent can have a boiling point of up to 340°C, 330°C, 320°C, 310°C, 300 °C, 290 °C, 280 °C, 270 °C, 260 °C or 250 °C. All possible combinations (e.g. 170 to 340°C) of the above lower and upper limits are also preferred. Preferably, the temperature is determined at 1013 mbar. In this application a "boiling point of 110 to 350°C" also encompasses those organic solvents (b) that undergo decomposition in said temperature range. Further, the boiling point does not relate to a single temperature but can also refer to a temperature interval, for example when a mixture of organic solvents is used.

Preferably, the boiling point is determined according to Pharm. Eur. 6.0, Chapter 2.2.12.

In a preferred embodiment of the composition the organic solvent (b) has a melting point between -80°C and 25°C, preferably between -75°C and 10°C, more preferably between -72°C and 5°C, especially between -70° and -5°C at 1013 mbar. Thus, the organic solvent used in the present invention is liquid at room temperature (23°C).

In a further embodiment of the invention the organic solvent (b) has a density of 0.95 to 1.30 g/ml.

It is further preferred that the organic solvent (b) has a density of 0.97 to 1.25 g/ml at 25°C, even more preferably 1.00 to 1.20 g/ml, especially 1.01 to 1.15 g/ml. The density can be determined the following formula:

$$\rho = \frac{m}{V}$$

m = the mass of the solvent
V = volume of the solvent

In a further embodiment of the invention the organic solvent (b) has a vapour pressure of less than 10 hPa or mbar at 20°C, preferably less than 1 hPa or mbar at 20°C.

The vapour pressure is the vapour exerted by a vapour P in a thermodynamic equilibrium with its liquid phase at a given temperature in a closed system. According to the Antoine equation the estimated vapour pressures can be calculated.

$$\log P = A - \frac{B}{C + T}$$

wherein A, B and C are substance-specific coefficients (i.e. constants or parameters) and
T is the temperature of the liquid.

For example, organic solvent (b) can be polyethylene glycols such as tetraethylene glycol and pentaethylene glycol, alcohol, polyethyleneglycol ether such as diethylene glycol monoethyl ether, glycerol, propylene glycol such as 1,2-propylene glycol, alkyl diols such as 2,3-butanediol, alkyl triols such as 1,2,6-hexantriol, isosorbide dimethyl ether, glycofurol (tetrahydrofufuryl alcohol polyethyleneglycol ether), polydimethyl siloxane, and mixtures thereof.

Particularly preferred as organic solvent (b) is glycofurol. Alternatively particularly preferred as organic solvent (b) is diethylene glycol monoethyl ether.

The composition of the present invention can preferably have a weight ratio of dissolved cebranopadol (a) to organic solvent (b) of 1:1 to 1:400, preferably of 1:2 to 1:150, more preferably of 1:3 to 1:100, even more preferably 1:4 to 1:80, most preferably of 1:5 to 1:60.

It turned out that with the above ratio it can be particularly assured that the complete amount of cebranopadol remains in the dissolved state and does not precipitate as a solid (crystalline) substance.

The present composition further comprises a carrier (c). The carrier is preferably solid, wherein "solid" refers to the appearance at 25°C. The term "carrier (c)" may refer to a single carrier (c) or a mixture of more than one carrier (c). Carrier (c) can be regarded as a substance to which dissolved cebranopadol (a) and organic solvent (b) can be adhered/adsorbed, wherein it is assured that cebranopadol maintains its dissolved state. Thus, carrier (c) can be regarded as a stabilizer of dissolved cebranopadol (a) in organic solvent (b). Generally, solid carrier (c) can be a substance which is capable of inhibiting the transformation of dissolved cebranopadol to any solid state (e.g. amorphous or crystalline) of cebranopadol.

Further, due to solid carrier (c) the physical composition can be provided in a state suitable for further processing, such as filling into a capsule.

In a preferred embodiment the composition of the invention can comprise cebranopadol (a) and carrier (c), wherein the weight ratio of (dissolved) cebranopadol (a) to carrier (c) can be from 1:1 to 1:150, preferably from 1:1 to 1:135, more preferably from 1:1 to 1:120, even more preferably from 1:1 to 1:110, and particularly from 1:1 to 1:100.

Generally, the carrier (c) can be a non-brittle or brittle substance.

Pharmaceutical excipients, such as carriers, can generally be classified with regard to the change in the shape of the particles under compression pressure (compaction): plastic excipients are characterised by plastic deformation, whereas when compressive force is exerted on brittle substances, the particles tend to break into smaller particles. Brittle behaviour on the part of the substrate can be quantified by the increase in the surface area in a moulding. In the art, it is customary to classify the brittleness in terms of the "yield pressure". According to a simple classification, the values for the "yield pressure" are low for plastic substances but high in the case of friable substances (Duberg, M., Nyström, C., 1982, "Studies on direct compression of tablets VI. Evaluation of methods for the estimation of particle fragmentation during compaction", Acta Pharm. Suec. 19, 421-436; Humbert-Droz P., Mordier D., Doelker E., "Méthode rapide de determination du comportement a la compression pour des études de preformulation.", Pharm. Acta Helv., 57, 136-143 (1982)). The "yield pressure" describes the pressure that has to be reached for the excipient (i.e. preferably the vehicle) to begin to flow plastically.

The "yield pressure" is preferably calculated by using the reciprocal of the gradient of the Heckel plot, as described in York, P., Drug Dev. Ind. Pharm. 18, 677 (1992). The measurement in this case is preferably made at 25°C and at a deformation rate of 0.1 mm/s.

In the context of the present invention, an excipient (especially a carrier) is deemed to be a non-brittle excipient when it has a "yield pressure" of not more than 120 MPa, preferably not more than 100 MPa, in particular 5 to 80 MPa. An excipient is usually described as a brittle excipient when it has a "yield pressure" of more than 80 MPa, preferably more than 100 MPa, particularly preferably more than 120 MPa, especially more than 150 MPa. Brittle excipients may exhibit a "yield pressure" of up to 300 MPa or up to 400 MPa or even up to 500 MPa.

Examples of non-brittle excipients (vehicles) are mannitol or starch.

Examples of brittle excipients (vehicles) are silicates or aluminosilicates, preferably magnesium aluminosilicates.

In a particularly preferred embodiment, brittle substances are used as carrier (c) in the oral dosage form of the present invention.

It is further preferred that the carrier (c) is a water insoluble substance. A water insoluble substance generally is a pharmaceutical excipient as specified in the European Pharmacopoeia with a water solubility of less than 33 mg/ml measured at 25°C. Preferably, the water insoluble substance has a solubility of 10 mg/ml or less, more preferably 5 mg/ml or less, especially 0.1 mg/ml, which means practically insoluble (determined according to the column elution method pursuant to EU Directive RL67-548-EWG, Appendix V Chapt. A6).

In a preferred embodiment of the invention the carrier can be an organic polymer or an inorganic substance.

In a preferred alternative embodiment of the invention the carrier (c) can preferably be an organic polymer. In

addition, the carrier (c) can also include substances which behave like polymers. Examples of these substances are fats and waxes. Furthermore, the carrier (c) can also include solid, non-polymeric compounds which preferably can contain polar side groups. Examples of these compounds are sugar alcohols or disaccharides.

In a preferred embodiment the carrier (c) can be a polymer. The polymer to be used for the preparation of the pharmaceutical composition preferably may have a glass transition temperature (Tg) of more than 45°C, more preferably of 50°C to 150°C, in particular of 55°C to 120°C. A respective Tg can be important for achieving the desired properties of the resulting dosage form.

In the present invention the term "glass transition temperature" (Tg) describes the temperature at which amorphous or partially crystalline polymers change from the solid state to the liquid state. In the process a distinct change in physical parameters, e.g. hardness and elasticity, occurs. Beneath the Tg a polymer is usually glassy and hard, whereas above the Tg it changes into a rubber-like to viscous state. The glass transition temperature is determined in the context of this invention by means of differential scanning calorimetry (DSC).

For this purpose, a Mettler Toledo® DSC 1 apparatus can be used. The work is performed at a heating rate of 1-20°C/min, preferably 10°C/min, and at a cooling rate of 5°C to 50°C/min, preferably 50°C/min.

In general, the organic polymer to be used as carrier (c) preferably can have a weight-average molecular weight of 1,000 to 500,000 g/mol, more preferably from 1,500 to 100,000 g/mol and particularly from 2,000 to 50,000 g/mol. The weight-average molecular weight is preferably determined by means of gel permeation chromatography.

In the present invention, hydrophilic polymers can preferably be used as carrier (c). The term "hydrophilic polymers" generally refers to polymers which possess hydrophilic groups. Examples of suitable hydrophilic groups can be hydroxy, sulfonate, carboxylate and quaternary ammonium groups.

Carrier (c) may, for example, comprise the following polymers: microcrystalline cellulose, polysaccharides, such as hydroxypropyl methyl cellulose (HPMC), ethyl cellulose, methyl cellulose, hydroxyethyl cellulose, ethyl hydroxyethyl cellulose, hydroxypropyl cellulose (HPC), polyvinyl alcohol, and mixtures thereof.

Alternatively, also a silicone, preferably further mixed with silicon dioxide such as simethicone, can be used as carrier (c).

In a more preferred embodiment of the invention carrier (c) can be an inorganic substance. An inorganic substance can preferably be regarded as a compound that does not contain a hydrocarbon group. It is further preferred that carrier (c) can be a phosphate or a silicate, preferably a silicate, more preferably an aluminosilicate.

Examples for inorganic substances suitable to be used as carriers are phosphates, such dicalcium phosphate, amorphous silica such as aerosol and silica gel, clay minerals such as kaolinite, bentonite and montmorillonite, kieselguhr (celite), zeolites, mesoporous silica such as Aeroperl® 300, MSU-G, MSU-F, Al-MCM-41, MCM-48, SBA-15 and SBA-16, magnesium aluminosilicates such as $Al_2O_3 \cdot MgO \cdot 1.7SiO_2 \cdot xH_2O$ (Neusilin®), and aluminosilicates such as Al-MCM-41 or mixtures thereof.

Further, active coal (i.e. activated carbon) can be preferably used as carrier (c).

In a preferred embodiment, carrier (c), in particular the inorganic carrier (c), has a specific surface area of 50 to 450 $m^2/g$, more preferably 75 to 400 $m^2/g$, in particular 100 to 300 $m^2/g$. The specific surface area preferably is determined by gas adsorption according to Ph. Eur., 6th edition, Chapter 2.9.26. For this purpose, an ASAP® 2020 (Micrometrics) and an 'outgasing' temperature of 40°C is used. It has surprisingly been found that the above-mentioned specific surface area might be beneficial for achieving the above-mentioned objects (e.g. stabilisation of the dissolved state of cebranopadol).

In a preferred embodiment, carrier (c) is selected from simethicone, activated carbon, microcrystalline cellulose, starch, polysaccharides, sugar alcohols, phosphates, silicon dioxides, clay minerals, kieselguhr (celite), zeolites, mesoporous silica and magnesium aluminosilicates, or mixtures thereof.

In a more preferred embodiment, carrier (c) is selected from simethicone, active coal, microcrystalline cellulose, phosphates, silicon dioxides, clay minerals, kieselguhr, zeolites, mesoporous silica and magnesium aluminosilicates, or mixtures thereof.

Most preferred as carrier (c) are silicates, in particular magnesium aluminosilicates, especially $Al_2O_3 \cdot MgO \cdot 1.7SiO_2 \cdot xH_2O$.

Alternatively most preferred as carrier (c) is silica, in particular mesoporous silica.

It is further preferred that the mesoporous silica is in form of pearl-like mesoporous granulates. The granulates can preferably have a mean particle size $D_{50}$ of 5 to 250 $\mu m$, more preferably 10 to 100 $\mu m$, even more preferably 15 to 80 $\mu m$, in particular 20 to 50 $\mu m$.

Preferably, the mesoporous silica has a specific surface area of 250 to 350 $m^2/g$, in particular, 280 to 320 $m^2/g$. Preferably, the volume of the mesoporous silica is 1.0 to 2.5 ml/g, more preferably 1.4 to 1.8 ml/g.

The mean particle size can refer to the $D_{50}$ value of the particle size distribution. The average particle size can be determined by means of laser diffractometry. In particular, a Malvern Instruments Mastersizer 2000 can be used to determine the size (preferably wet measurement with ultrasound 60 sec., 2,000 rpm, preferably dispersed in sunflower oil, the evaluation being performed according to Particle RI set to 1.520 and Absorption of 2).

In a particularly preferred embodiment Aeroperl® 300 is used.

In an alternative particularly preferred embodiment Neusilin® is used.

In a preferred embodiment the composition of the present invention can preferably comprise the following amounts of components:

0.5 to 1000 μg cebranopadol, preferably 0.8 to 800 μg cebranopadol, more preferably 5 to 600 μg cebranopadol, particularly 10 to 400 μg, especially 200 μg cebranopadol
0.01 to 150 mg organic solvent, preferably 0.02 to 100 mg organic solvent, particularly 0.1 to 80 mg organic solvent,
0.005 to 150 mg carrier, preferably 0.01 to 120 mg carrier, particularly 0.05 to 90 mg carrier.

The composition of the present invention can be applied in form of an oral dosage form, in particular in form of a solid oral dosage form. Thus, another object of the present invention is a solid oral dosage form comprising a cebranopadol composition according to the present invention and further pharmaceutical excipient(s).

The pharmaceutical excipients are excipients with which the person skilled in the art is familiar, such as those which are described in the European Pharmacopoeia (Ph.Eur.) and/or in the US Pharmacopoeia (USP).

In a preferred embodiment of the present invention the oral dosage form can further comprise one or more excipients(s) selected from surfactants (d), fillers (e), binders (f), disintegrants (g), lubricants (h), and glidants (j).

Surfactants (d) can be regarded as substances lowering the interfacial tension between two phases, thus enabling or supporting the formation of dispersions or working as a solubilizer. Common surfactants are alkylsulfates (for example sodium lauryl sulfate), alkyltrimethylammonium salts, alcohol ethoxylates and the like. Surfactants can be used in an amount of 0 to 2% by weight, preferably of 0.1 to 1.5% by weight, based on the total weight of the oral dosage form.

It is particularly preferred that the oral dosage form of the present invention does not contain a surfactant.

Fillers (e) or diluents can be used to increase the bulk volume and weight of a low-dose drug to a limit at which a pharmaceutical dosage form can be formed. Fillers should fulfil several requirements, such as being chemically inert, non-hygroscopic, biocompatible, easily processable and possessing good biopharmaceutical properties. Examples of fillers are lactose, sucrose, glucose, mannitol, calcium carbonate, cellulose and others. Fillers (e) can be used in an amount of 0 to 75% by weight, more preferably 5 to 40% by weight, based on the total weight of the dosage form.

Binders (f) may be added to the pharmaceutical formulation in order to ensure that oral dosage forms, preferably tablets, can be formed with the required mechanical strength. The binder can, for example, be starch, polyvinyl pyrrolidone or cellulose derivatives. The binding agent can be present in an amount of 0 to 20% by weight, preferably 1 to 18% by weight, more preferably 2 to 15% by weight, in particular 3 to 12% by weight, based on the total weight of the pharmaceutical formulation.

Disintegrants (g) are compounds which enhance the ability of the dosage form, preferably the ability of the tablet to break into smaller fragments when in contact with a liquid, preferably water. Preferred disintegrants are sodium carboxymethyl starch, cross-linked polyvinyl pyrrolidone (crospovidone), sodium carboxymethyl glycolate (for example Explotab®), swelling polysaccharide, for example soy polysaccharide, carrageenan, agar, pectin, starch and derivatives thereof, protein, for example formaldehyde-casein, sodium bicarbonate, or mixtures thereof. More preferred are sodium carboxymethyl cellulose and cross-linked polyvinyl pyrrolidone (crospovidone). Disintegrants can be used in an amount of 0 to 15% by weight, preferably of 1 to 12% by weight, more preferably 3 to 10% by weight, based on the total weight of the dosage form.

The function of lubricants (h) is reported to ensure that tablet formation and ejection can occur with low friction between the solid and the die wall. Further, lubricants can generally increase the powder flowability. The lubricant is preferably a stearate or fatty acid, more preferably an earth alkali metal stearate, such as magnesium stearate. The lubricant is suitably present in an amount of 0 to 2% by weight, preferably of about 0.1 to 1.0% by weight, based on the total weight of the dosage form.

Glidants (j) can also be used to improve the flowability. Traditionally, talc was used as glidant, but is nowadays nearly fully replaced by colloidal silica (for example Aerosil® Preferably, the glidant can be present in an amount of 0 to 3% by weight, more preferably 0.1 to 2.5% by weight, in particular 0.25 to 2.0% by weight based on the total weight of the dosage form.

It lies in the nature of pharmaceutical excipients that they sometimes can perform more than one function in a pharmaceutical formulation. In this regard it is generally noted that due to the nature of pharmaceutical excipients it cannot be excluded that a certain compound meets the requirements of more than one of the components (b) or (c) and (d) to (j). Therefore, colloidal silica (Aerosil) may function as a carrier for forming the composition according to the invention as well as a glidant (j), i.e. the fact that colloidal silica is used as component (c) for forming the composition according to the invention does not mean that it cannot also be acting as a glidant (j).

However, in order to enable an unambiguous distinction, it is preferred in the present application that one and the same pharmaceutical compound can only function as one of the compounds (b) or (c) and (d) to (j). For example, if

microcrystalline cellulose functions as a carrier (c), it cannot additionally function as a disintegrant (g), even though microcrystalline cellulose also exhibits a certain disintegrating effect.

The oral dosage form of the present invention can preferably comprise the following amounts of components:

0.5 to 1000 μg cebranopadol, preferably 0.8 to 800 μg cebranopadol, more preferably 5 to 600 μg cebranopadol, particularly 10 to 400 μg, especially 200 μg cebranopadol,
0.01 to 150 mg organic solvent, preferably 0.02 to 100 mg organic solvent, particularly 0.1 to 80 mg organic solvent,
0.005 to 150 mg carrier, preferably 0.01 to 120 mg carrier, particularly 0.05 to 90 mg carrier,
0 to 20 mg surfactant, preferably 2 to 15 mg surfactant, particularly 4 to 10 mg surfactant,
0 to 400 mg filler, preferably 25 to 300 mg filler, particularly 40 to 200 mg filler, 0 to 150 mg binder, preferably 15 to 100 mg binder, particularly 25 to 75 mg binder,
0 to 100 mg disintegrant, preferably 5 to 75 mg disintegrant, particularly 10 to 50 mg disintegrant,
0 to 15 mg lubricant, preferably 1 to 10 mg lubricant, particularly 2 to 8 mg lubricant,
0 to 25 mg glidant, preferably 1 to 15 mg glidant, particularly 2 to 7 mg glidant.

In a preferred embodiment the oral dosage form of the present invention can preferably comprise:

0.005 to 2 wt.‰ cebranopadol, preferably 0.01 to 1.5 wt. ‰ cebranopadol, particularly 0.02 to 1 wt. ‰ cebranopadol,
0.1 to 30 wt.% organic solvent, preferably 2 to 28 wt.% organic solvent, particularly 5 to 25 wt.% organic solvent,
0.1 to 28 wt.% carrier, preferably 2 to 26 wt.% carrier, particularly 5 to 24 wt.% carrier,
0 to 2 wt.% surfactant, preferably 0.05 to 1.6 wt.% surfactant, particularly 0.1 to 1.5 wt.% surfactant,
0 to 75 wt.% filler, preferably 3 to 65 wt.% filler, particularly 5 to 40 wt.% filler,
0 to 20 wt.% binder, preferably 2 to 15 wt.% binder, particularly 3 to 12 wt.% binder,
0 to 15 wt.% disintegrant, preferably 1 to 12 wt.% disintegrant, particularly 3 to 10 wt.% disintegrant
0 to 2 wt.% lubricant, preferably 0.1 to 1.0 wt.% lubricant, particularly 0.2 to 0.8 wt.% lubricant,
0 to 3 wt.% glidant, preferably 0.1 to 2.5 wt.% glidant, particularly 0.25 to 2.0 wt.% glidant,
based on the total weight of the oral dosage form.

In a still further embodiment of the present invention the oral dosage form can be a capsule or a tablet, more preferably a tablet, for peroral use. Alternatively, the solid oral dosage form can be filled as powder or granulate into devices like sachets or stick-packs.

The present invention further relates to a method for producing a composition according to the invention. Hence, a further subject of the present invention is a method for producing a composition comprising dissolved cebranopadol (a), organic solvent (b) and carrier (c) comprising the steps of

i) dissolving cebranopadol in an organic solvent with a boiling point of 110° to 350°C (b),
ii) mixing carrier (c) and the solution of step i), and
iii) optionally milling and/or sieving the mixture of step ii)

Generally, the comments made above for cebranopadol, organic solvent and carrier can also apply to the method of the present invention.

In step i) of the method of the invention cebranopadol (a) is dissolved in organic solvent (b).

The term "dissolving" means that a substance, such as cebranopadol, is brought into contact with the solvent, preferably with a solvent or solvent mixture as defined above for compound (b), e.g. a glycofurol, wherein the solvent wets the surface of the substance or the substance can be completely dissolved in the solvent. When a clear solution is obtained and no cebranopadol (crystals) can be detected by visual control, this can be regarded as a complete dissolving of cebranopadol in an organic solvent.

In a preferred embodiment cebranopadol is preferably added to organic solvent. It is further preferred that the organic solvent is preferably stirred and/or heated, preferably to a temperature of about 80°C.

In a preferred embodiment cebranopadol (a) can be dissolved in organic solvent (b), preferably under stirring during the dissolving step, preferably at a stirring speed of 300 to 450 rpm (rotations per minute). Additionally, it is preferred that the solvent is at an elevated temperature, preferably at about 80°C, during the dissolving step. Further, cebranopadol is preferably added in crystalline form.

Further, to support the formation of the solution of step i) cebranopadol in organic solvent can be subjected to a mechanical treatment, such as ultrasonic treatment. Generally, ultrasonic treatment can be carried out by immersing cebranopadol and organic solvent into an ultrasonic device, for example an ultrasonic bath. Examples of ultrasonic treatment are hydrodynamic cavitation, sono-fragmentation and/or sono-cavitation or co-grinding. For example, ultrasonic treatment can be carried out with Tesla ultrasonic equipment.

Ultrasonic treatment can preferably be performed by using ultrasonic waves having a frequency of 5 to 100 kHz, more preferably of 10 to 80 kHz. Furthermore, ultrasonic treatment is preferably performed by using ultrasonic waves having an intensity of 50 to 5000 W, more preferably 500 to 1000 W. As an example, 1000 W and 20 kHz or 500 W and 58 kHz can be used.

Usually, the mechanical treatment can be carried out for 1 to 30 minutes, preferably for 5 to 20 minutes.

Once the cebranopadol is completely dissolved in organic solvent (b) in step ii), carrier (c) and the solution of step i) can be mixed.

In a preferred embodiment carrier (c) is added to the solution of step i). It is preferred that the solution of step i) is at elevated temperature, preferably about 80°C, when the mixing with carrier (c) is carried out. Further, the solution is preferably stirred, preferably at a stirring speed of 300 to 550 rpm (rotations per minute) during the mixing step ii).

In a preferred embodiment the mixture of step ii), preferably after being allowed to cool to 23°C, can be obtained as a powder-like material.

In an alternative embodiment further pharmaceutical active agent can be added to the mixture between step ii) and optional step iii).

In optional step iii) the mixture of step ii) can preferably be milled and/or sieved.

The milling can preferably be performed in conventional milling apparatuses, such as in a ball mill, air jet mill, pin mill, classifier mill, cross beater mill, disk mill, mortar grinder or a rotor mill. A planetary ball mill is preferably used.

The milling time is preferably 0.5 minutes to 30 minutes, preferably 1 to 15 minutes, more preferably 3 to 7 minutes.

It is preferred that the sieving of the mixture of step ii) can be carried out with a sieve having a mesh size of 25 to 1000 μm, preferably 50 to 800 μm, especially 100 to 600 μm.

Further, the subject of the present invention relates to a method for preparing the oral dosage of the invention comprising the steps of:

i) dissolving cebranopadol in organic solvent with a boiling point of 110° to 350°C (b),
ii) mixing carrier (c) and the solution of step i),
iii) optionally milling and/or sieving the mixture of step ii),
iv) optionally adding further excipient(s) to the mixture of step ii) or step iii),
v) optionally granulating the mixture of step ii), step iii) or step iv)
vi) processing the mixture of step ii), step iii) or step iv) or the granulates of step v) into an oral dosage form.

In steps i) to iii) a composition according to the present invention is provided, i.e. all the above process steps i), ii) and iii) leading to the present composition also apply to the process for preparing the present oral dosage form.

In step iv) additional further excipient(s) and/or further pharmaceutically active agent(s) can optionally be added to the mixture of step ii) or step iii). During or after the addition of the optional excipients and/or further pharmaceutically active agent the resulting mixture can preferably be blended. The excipients can preferably be selected from excipients (d), (e), (f), (g), (h) and (j) as described above.

In step v) the mixture of step ii), step iii) or step iv) can optionally be granulated.

"Granulating" is generally understood to mean the formation of relatively coarse or granular aggregate material as a powder by assembling and/or aggregating finer powder particles (agglomerate formation or build-up granulation) and/or the formation of finer granules by breaking up coarser aggregates (disintegration or break-down granulation).

Granulation can conventionally mean wet or dry granulation.

Dry granulation, which is preferred, is generally carried out by using pressure. In a preferred embodiment of the invention, granulating the mixture of step iv) can be performed, for example, by "slugging", using a large heavy-duty rotary press and breaking up the slugs into granulates with a hammer mill or by roller compaction, using for example roller compactors by Powtec or Alexanderwerk. The granulates are then optionally screened.

In step vi) the mixture of step ii), step iii), step iv) or the granulates of step v) is/are processed into a solid oral dosage form. Processing the mixture of step ii), step iii), or step iv) or the granulates of step v) into a solid oral dosage form can preferably comprise filling said mixture into capsules, preferably hard gelatine capsules. Optionally, processing the mixture of step ii), step iii) or step iv) or the granulates of step v) into tablets can be carried out by compressing said formulation on a rotary press, e.g. on a Fette® (Fette GmbH, Germany) or a Riva® piccola (Riva, Argentina). The main compression force can range from 1 to 50 kN, preferably 3 to 40 kN. The resulting tablets can have a hardness of 30 to 400 N, more preferred of 50 to 250 N, particularly preferably of 30 to 180 N, more preferably 40 to 150 N, wherein the hardness can be measured according to Ph.Eur. 6.0, Chapter 2.9.8. For the optional filling of the formulation into capsules, dependent dosing systems (for example an auger) or preferably independent dosing systems (for example MG2, Matic (IMA)) can be used.

Further, the dosage form, preferably the tablet, of the invention preferably has a content uniformity, i.e. a content of active agent(s), which lies within the concentration of 90 to 110%, preferably 95 to 105%, especially preferred from

98 to 102% of the average content of the active agents(s). The "content uniformity" is determined with a test in accordance with Ph. Eur., 6.0, Chapter 2.9.6. According to that test, the content of active agent(s) of each individual tablet out of 20 tablets must lie between 90 and 110%, preferably between 95 and 105%, especially between 98 and 102% of the average content of the active agents(s). Therefore, the content of the active drugs in each tablet of the invention differs from the average content of the active agent by at most 10%, preferably at most 5% and especially at most 2%.

In addition, the resulting tablet preferably has a friability of less than 5%, particularly preferably less than 2%, especially less than 1%. The friability is determined in accordance with Ph. Eur., 6.0, Chapter 2.9.7. The friability of tablets generally refers to tablets without coating.

The pharmaceutical formulation of the invention may be a peroral tablet which can be swallowed unchewed. The tablet can preferably be film coated.

Generally, film coatings that do not affect the release of the active agent(s) and film coatings affecting the release of the active agent(s) can be employed with tablets according to invention. The film coatings that do not affect the release of the active agent(s) are preferred.

Preferred examples of film coatings which do not affect the release of the active ingredient can be those including poly(meth)acrylate, methylcellulose (MC), hydroxypropyl methylcellulose (HPMC), hydroxypropyl cellulose (HPC), hydroxyethyl cellulose (HEC), polyvinylpyrrolidone (PVP) and mixtures thereof. These polymers can have a weight-average molecular weight of 10,000 to 150,000 g/mol.

In an alternative preferred embodiment, the film coating can affect the release of the active agent. Examples for film coatings affecting the release of the active agent are gastric juice-resistant film coatings and retard coatings.

In a preferred embodiment the film can have a thickness of 2 $\mu$m to 150 $\mu$m, preferably from 10 to 100 $\mu$m, more preferably from 20 to 60 $\mu$m.

In a preferred embodiment the dosage form of the invention is for modified release. In that case the release profile of the pharmaceutical formulation, preferably of the tablet, according to USP method (USP paddle apparatus, 900 ml test medium, in phosphate buffer at pH 6.8 and 37°C, 100 rpm) after 2 hours indicates a content release of 0 to 90%, preferably of 10 to 80%, further preferably 15 to 75%, more preferably 20 to 50% and particularly of 25 to 40%.

In an alternatively preferred embodiment of the invention the dosage form is for immediate release. In that case the release profile of the pharmaceutical formulation, preferably of the tablet, according to USP method (USP paddle apparatus, 900 ml test medium, in phosphate buffer at pH 6.8 and 37°C, 100 rpm) after 15 minutes indicates a content release of at least 50%, preferably at least 70%, especially at least 90%.

Further, the invention relates to a method for treating chronic pain including neuropathic pain, as such in patients with tumor(s), comprising administering to a subject in need thereof a therapeutically effective amount of a pharmaceutical composition comprising (a) dissolved cebranopadol, (b) organic solvent with a boiling point of about 110°C to 350°C, and (c) solid carrier. For the pharmaceutical composition administered in the before-mentioned method the same applies as to the composition as described above in the text.

**Experimental Part**

**Analytical Methods:**

The compositions according to the invention were examined by X-ray powder diffraction.

**X-ray powder diffraction**

The measurements were performed as follows: The samples were measured on a D8 Advance powder X-ray diffractometer (Bruker-AXS, Karlsruhe, Germany) in a PMMA sample holder (diameter: 25 m; depth: 1 mm) rotating at 20 rpm during the measurement in reflection mode (Bragg-Brentano geometry). Further conditions for the measurements are summarized below. The raw data were analysed with the program EVA (Bruker-AXS, Karlsruhe, Germany). Background subtraction and K$\alpha_2$ stripping were not performed.

| | |
|---|---|
| radiation | Cu $K_{\alpha 1/\alpha 2}$ |
| source | 34kV/40mA |
| detector | Vantec-1 (electronic window: 3°) |
| K$\beta$ filter | Ni (diffracted beam) |
| measuring circle diameter | 435 mm |
| detector window slit | 12 mm |
| anti-scatter slit | 8 mm |
| divergence slit | v6.00 (variable) |

(continued)

| | |
|---|---|
| soller slit (incident/diffracted beam) | 2.5° |
| 2θ range | 2° ≤ 2θ ≤ 55° |
| step size | 0.016° |
| step time | 0.2 s |

## EXAMPLES

Cebranopadol was dissolved in solvent at 80°C. The carrier material was added portion wise (0.05 g) at 80°C until the mixture was a free flowing homogenous powder. The composition was allowed to cool to room temperature (23°C).

The employed amounts and the resulting solid state of the formulations are specified in Table 1.

**Table 1:** Composition and solid state analysis of Cebranopadol formulations

| Example no. | Solvent | Solvent [ml] | Cebranopadol [mg] | Carrier | Carrier [mg] |
|---|---|---|---|---|---|
| 1 | glycofurol | 0.4 | 10 | Neusilin® | 200 |
| 2 | glycofurol | 0.4 | 10 | SBA-16 | 650 |
| 3 | glycofurol | 0.4 | 10 | SBA-15 | 200 |
| 4 | glycofurol | 0.4 | 10 | MSU-F | 150 |
| 5 | glycofurol | 0.4 | 10 | MCM-48 | 200 |
| 6 | glycofurol | 0.4 | 10 | Al-MCM-41 | 200 |
| 7 | glycofurol | 0.4 | 10 | Celite | 400 |
| 8 | glycofurol | 0.4 | 10 | Aeroperl® 300 | 200 |
| 9 | diethylene glycol monomethyl ether | 0.55 | 10 | Neusilin® | 250 |
| 10 | diethylene glycol monomethyl ether | 0.55 | 10 | SBA-16 | 950 |
| 11 | diethylene glycol monomethyl ether | 0.55 | 10 | SBA-15 | 250 |
| 12 | diethylene glycol monomethyl ether | 0.55 | 10 | MSU-F | 250 |
| 13 | diethylene glycol monomethyl ether | 0.55 | 10 | MCM-48 | 250 |
| 14 | diethylene glycol monomethyl ether | 0.55 | 10 | Al-MCM-41 | 250 |
| 15 | diethylene glycol monomethyl ether | 0.55 | 10 | Celite | 400 |
| 16 | diethylene glycol monomethyl ether | 0.55 | 10 | Aeroperl® 300 | 250 |

## RESULTS

Figure A-1 shows an overview of X-ray powder diffractograms of all used carriers.

In Figure A-2 the diffractogram of the Example 1 is shown. The diffractogram essentially looks like the diffractogram of pure Neusilin® (compare with Figure A-1)·

In Figure A-3 the diffractogram of Example 2 is shown. Again, the diffractogram essentially looks like the diffractogram of pure SBA-16 (compare with Figure A-1).

In Figure A-4 the diffractogram of Example 3 is shown. The diffractogram essentially looks like the diffractogram of pure SBA-15 (compare with Figure A-1).

In Figure A-5 the diffractogram of Example 4 is shown. The diffractogram essentially looks like the diffractogram of pure MSU-F (compare with Figure A-1).

In Figure A-6 the diffractogram of Example 5 is shown. The diffractogram essentially looks like the diffractogram of pure MCM-48 (compare with Figure A-1).

In Figure A-7 the diffractogram of Example 6 is shown. The diffractogram essentially looks like the diffractogram of pure Al-MCM-41 (compare with Figure A-1).

In Figure A-8 the diffractogram of Example 7 is shown. The diffractogram essentially looks like the diffractogram of pure Celite (compare with Figure A-1).

In Figure A-9 the diffractogram of Example 8 is shown. The diffractogram essentially looks like the diffractogram of pure Aeroperl® 300 (compare with Figure A-1).

The organic solvent glycofurol (polyethyleneglycol tetrahydrofurfuryl ether) has a boiling point of 100-145°C at 0.4 Hg. Although a high possible concentration of cebranopadol in solution was obtained, there is no indication that cebranopadol crystallises in the present sample, i.e. cebranopadol stays dissolved.

In Figure A-10 the diffractogram of the Example 9 is shown. The diffractogram essentially looks like the diffractogram of pure Neusilin® (compare with Figure A-1).

Similar results are obtained for the other examples 10 to 16.

The organic solvent diethylene glycol monoethyl ether has a boiling point of 202°C at atmospheric pressure. Although a high possible concentration of cebranopadol in solution was obtained, there is no indication that cebranopadol crystallises in the present sample, i.e. cebranopadol stays dissolved.

## Claims

1. Pharmaceutical composition comprising

   (a) dissolved cebranopadol,
   (b) organic solvent with a boiling point of 110° to 350° C, and
   (c) solid carrier.

2. Composition according to claim 1, wherein the organic solvent (b) has a boiling point of 170°C to 345°C.

3. Composition according to claim 1 or 2, wherein the organic solvent (b) has a density of 0.95 to 1.30 g/ml, measured at 20°C.

4. Composition according to any one of claims 1 to 3, wherein the weight ratio of dissolved cebranopadol (a) to organic solvent (b) is from 1:1 to 1:150.

5. Composition according to any one of claims 1 to 4, wherein the carrier (c) is a brittle substance, having a yield pressure of 80 MPa to 500 MPa.

6. Composition according to any one of claims 1 to 5, wherein the carrier (c) is an organic polymer or an inorganic substance.

7. Composition according to any one of claims 1 to 6, wherein the carrier (c) possesses a specific surface area from 75 to 350 $m^2/g$, whereby the specific surface area is measured according to Ph.Eur. 6.0, 2.9.26.

**8.** Composition according to any one of claims 1 to 7, wherein the carrier (c) is a silicate, preferably a magnesium aluminosilicate, or mesoporous silica.

**9.** Composition according to any one of claims 1 to 8, wherein the weight ratio of dissolved cebranopadol (a) to carrier (c) is from 1:1 to 1:100.

**10.** Oral dosage form comprising a composition according to any one of claims 1 to 9 and optionally further pharmaceutical excipient(s).

**11.** Oral dosage form according to claim 10, wherein the dosage form comprises
0.005 to 2 wt.‰ cebranopadol, preferably 0.01 to 1.5 wt.‰ cebranopadol, particularly
0.02 to 1 wt.‰ cebranopadol,
0,1 to 30 wt.% organic solvent, preferably 2 to 28 wt.% organic solvent,
0,1 to 28 wt.% carrier, preferably 2 to 26 wt.% carrier,
0 to 2 wt.% surfactant, preferably 0.05 to 1.6 wt.% surfactant,
0 to 75 wt.% filler, preferably 3 to 65 wt.% filler,
0 to 20 wt.% binder, preferably 2 to 15 wt.% binder,
0 to 15 wt.% disintegrant, preferably 1 to 12 wt.% disintegrant,
0 to 2 wt.% lubricant, preferably 0.1 to 1.0 wt.% lubricant,
0 to 3 wt.% glidant, preferably 0.1 to 2.5 wt.% glidant,
based on the total weight of the oral dosage form.

**12.** Method for producing a composition according to any one of claims 1 to 9 comprising the steps of

i) dissolving cebranopadol (a) in organic solvent with a boiling point of 110° to 350°C (b)
ii) mixing carrier (c) and the solution of step i)
iii) optionally milling and/or sieving the mixture of step ii).

**13.** Method for producing an oral dosage form according to claim 10 or 11 comprising the steps of

i) dissolving cebranopadol (a) in organic solvent with a boiling point of 110° to 350°C (b)
ii) mixing carrier (c) and the solution of step i)
iii) optionally milling and/or sieving the mixture of step ii)
iv) optionally adding further excipient(s) to the mixture of step ii) or step iii),
v) optionally granulating the mixture of step ii), step iii) or step iv)
vi) processing the mixture of step ii), step iii) or step iv) or the granulates of step v) into an oral dosage form.

**14.** A composition according to claim 1 for use in treating chronic pain including neuropathic pain, preferably in patients with tumor(s).

**Patentansprüche**

**1.** Pharmazeutische Zusammensetzung umfassend

(a) gelöstes Cebranopadol,
(b) organisches Lösungsmittel mit einem Siedepunkt von 110° bis 350°C und
(c) festen Trägerstoff.

**2.** Zusammensetzung gemäß Anspruch 1, wobei das organische Lösungsmittel (b) einen Siedepunkt von 170°C bis 345°C hat.

**3.** Zusammensetzung gemäß einem der Ansprüche 1 oder 2, wobei das organische Lösungsmittel (b) eine Dichte von 0,95 bis 1,30 g/ml aufweist, gemessen bei 20°C.

**4.** Zusammensetzung gemäß einem der Ansprüche 1 bis 3, wobei das Gewichtsverhältnis des gelösten Cebranopadols (a) zum organischen Lösungsmittel (b) von 1:1 bis 1:150 beträgt.

5. Zusammensetzung gemäß einem der Ansprüche 1 bis 4, wobei der Trägerstoff (c) eine brüchige Substanz ist, die einen Nenndruck von 80 MPa bis 500 MPa aufweist.

6. Zusammensetzung gemäß einem der Ansprüche 1 bis 5, wobei der Trägerstoff (c) ein organisches Polymer oder eine anorganische Substanz ist.

7. Zusammensetzung gemäß einem der Ansprüche 1 bis 6, wobei der Trägerstoff (c) eine spezifische Oberfläche von 75-350 $m^2/g$ aufweist, wobei die spezifische Oberfläche gemäß Ph.Eur. 6.0, 2.9.26 gemessen wird.

8. Zusammensetzung gemäß einem der Ansprüche 1 bis 7, wobei der Trägerstoff (c) ein Silikat ist, bevorzugt ein Magnesiumaluminosilikat oder mesoporöse Kieselerde.

9. Zusammensetzung gemäß einem der Ansprüche 1 bis 8, wobei das Gewichtsverhältnis des gelösten Cebranopadol (a) zum Trägerstoff (c) von 1:1 bis 1:100 beträgt.

10. Orale Darreichungsform umfassend eine Zusammensetzung gemäß einem der Ansprüche 1 bis 9 und optional weiter(e) pharmazeutische(s) Hilfsstoff(e).

11. Orale Darreichungsform gemäß Anspruch 10, wobei die Darreichungsform umfasst
0,05 bis 2 Gew.% Cebranopadol, bevorzugt 0,01 bis 1,5 Gew.% Cebranopadol, insbesondere 0,02 bis 1 Gew.% Cebranopadol,
0,1 bis 30 Gew.% organisches Lösungsmittel, bevorzugt 2 bis 28 Gew.% organisches Lösungsmittel,
0,1 bis 28 Gew.% Trägerstoff, bevorzugt 2 bis 26 Gew.% Trägerstoff,
0 bis 2 Gew.% Detergens, bevorzugt 0,05 bis 1,6 Gew.% Detergens,
0 bis 75 Gew.% Füllstoff, bevorzugt 3 bis 65 Gew.% Füllstoff,
0 bis 20 Gew.% Bindemittel, bevorzugt 2 bis 15 Gew.% Bindemittel,
0 bis 15 Gew.% Sprengmittel, bevorzugt 1 bis 12 Gew.% Sprengmittel,
0 bis 2 Gew.% Gleitmittel, bevorzugt 0,1 bis 1,0 Gew.% Gleitmittel,
0 bis 3 Gew.% Schmiermittel, bevorzugt 0,1 bis 2,5 Gew.% Schmiermittel,
bezogen auf das Gesamtgewicht der oralen Darreichungsform.

12. Verfahren zur Herstellung einer Zusammensetzung gemäß einem der Ansprüche 1 bis 9, umfassend die Schritte

(i) Lösung von Cebranopadol (a) in einem organischen Lösungsmittel mit einem Siedepunkt von 110° bis 350°C (b),
(ii) Mischen von Trägerstoff (c) und der Lösung aus Schritt (i),
(iii) optional Vermahlen und /oder Sieben der Mischung aus Schritt (ii).

13. Verfahren zur Herstellung einer oralen Darreichungsform gemäß Anspruch 10 oder 11, umfassend die Schritte

i) Lösen von Cebranopadol (a) in einem organischen Lösungsmittel mit einem Siedepunkt von 110° bis 350°C (b),
ii) Mischen von Trägerstoff (c) und der Lösung aus Schritt (i),
iii) optional Vermahlen und /oder Sieben der Mischung aus Schritt (ii),
iv) optional Hinzufügen weiterer Hilfsstoff(e) zur Mischung aus Schritt ii) oder iii),
v) optional Granulieren der Mischung aus Schritt ii), Schritt iii) oder Schritt iv),
vi) Verarbeiten der Mischung aus Schritt ii), Schritt iii) oder Schritt iv) oder des Granulats aus Schritt v) in eine orale Darreichungsform.

14. Zusammensetzung gemäß Anspruch 1 zur Verwendung in der Behandlung von chronischen Schmerzen, einschließlich neuropathischen Schmerz, bevorzugt in Patienten mit Tumor(en).

## Revendications

1. Composition pharmaceutique comprenant

(a) cébranopadol dissolu,
(b) un solvant organique ayant un point d'ébullition de 110 à 350°C et

(c) un support solide.

**2.** Composition selon la revendication 1 dans laquelle le solvant (b) a un point d'ébullition de 170 à 345°C.

**3.** Composition selon l'une quelconque des revendications 1 ou 2 dans laquelle le solvant organique (b) a une densité de 0,95 à 1,30 g/ml, mesurée à 20°C.

**4.** Composition selon l'une quelconque des revendications 1 à 3 dans laquelle le rapport pondéral du cébranopadol dissolu (a) au solvant organique (b) est de 1:1 à 1:150.

**5.** Composition selon l'une quelconque des revendications 1 à 4 dans laquelle le support (c) est une substance friable ayant une pression nominale de 80 MPa à 500 MPa.

**6.** Composition selon l'une quelconque des revendications 1 à 5 dans laquelle le support (c) est un polymère organique ou une substance inorganique.

**7.** Composition selon l'une quelconque des revendications 1 â 6 dans laquelle le support (c) possède une surface spécifique de 75 à 350 $m^2/g$, la surface spécifique étant mesurée selon Ph.Eur. 6.0, 2.9.26.

**8.** Composition selon l'une quelconque des revendications 1 à 7 dans laquelle le support (c) est un silicate, de préférence un aluminosilicate de magnésium ou de la silice mésoporeuse.

**9.** Composition selon l'une quelconque des revendications 1 à 8 dans laquelle le rapport pondéra du cébranopadol dissolu (a) au support (c) est de 1:1 à 1:100.

**10.** Forme posologique orale comprenant une composition selon l'une quelconque des revendications 1 à 9 et en option d'autre(s) excipient(s) pharmaceutique(s).

**11.** Forme posologique orale selon la revendication 10, la forme posologique comprenant
0,005 à 2 % en poids de cébranopadol, de préférence 0,01 à 1,5 % en poids de cébranopadol, en particulier 0,02 à 1 % en poids de cébranopadol,
0,1 à 30 % en poids de solvant organique, de préférence 2 à 28% en poids de solvant organique,
0,1 à 28 % en poids de support, de préférence 2 à 26 % en poids de support,
0 à 2 % en poids de détergent, de préférence 0,05 à 1,6 % en poids de détergent,
0 à 75 % en poids de charge, de préférence 3 à 65 % en poids de charge,
0 à 20 % en poids de liant, de préférence 2 à 15 % en poids de liant,
0 à 15% en poids de désintégrant, de préférence 1 à 12 % en poids de désintégrant,
0 à 2 % en poids de lubrifiant, de préférence 0,1 à 1,0 % en poids de lubrifiant,
0 à 3 % en poids d'agent de glissement, de préférence 0,1 à 2,5 % en poids d'agent de glissement,
par rapport au poids total de la forme posologique orale.

**12.** Procédé pour la production d'une composition selon l'une quelconque des revendications 1 à 9 comprenant les étapes de

i) dissoudre du cébranopadol (a) dans du solvant organique ayant un point débullition de 110° à 350°C (b)
ii) mélanger le support (c) et la solution de l'étape i)
iii) en option moudre et/ou tamiser le mélange de l'étape ii).

**13.** Procédé pour la production d'une forme posologique orale selon la revendication 10 ou 11 comprenant les étapes de

i) dissoudre du cébranopadol (a) dans du solvant organique ayant un point débullition de 110° à 350°C (b)
ii) mélanger le support (c) et la solution de l'étape i)
iii) en option moudre et/ou tamiser le mélange de l'étape ii)
iv) en option ajouter d'autre(s) excipient(s) au mélange de l'étape ii) ou de l'étape iii),
v) en option granuler le mélange de l'étape ii), de l'étape iii) ou de l'étape iv)
vi) traiter le mélange de l'étape ii), de l'étape iii) ou de l'étape iv) ou le granulat de l'étape v) pour le transformer en forme posologique orale.

**14.** Composition selon la revendication 1 pour utilisation dans le traitement de la douleur chronique, y compris la névralgie, de préférence chez les patients avec (une) tumeur(s).

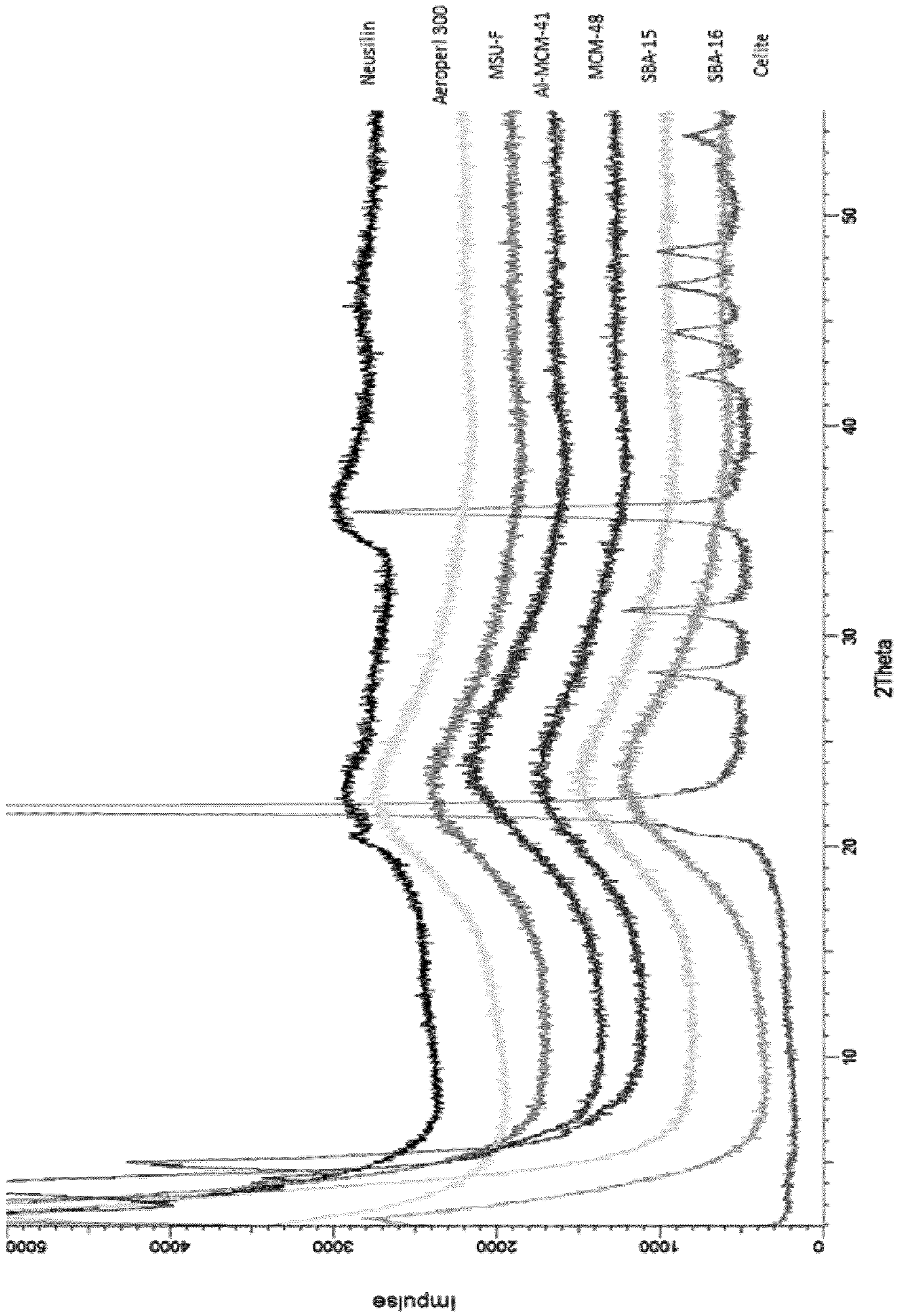


**Figure A-1: Overview of X-ray powder diffractograms of all used carriers.**

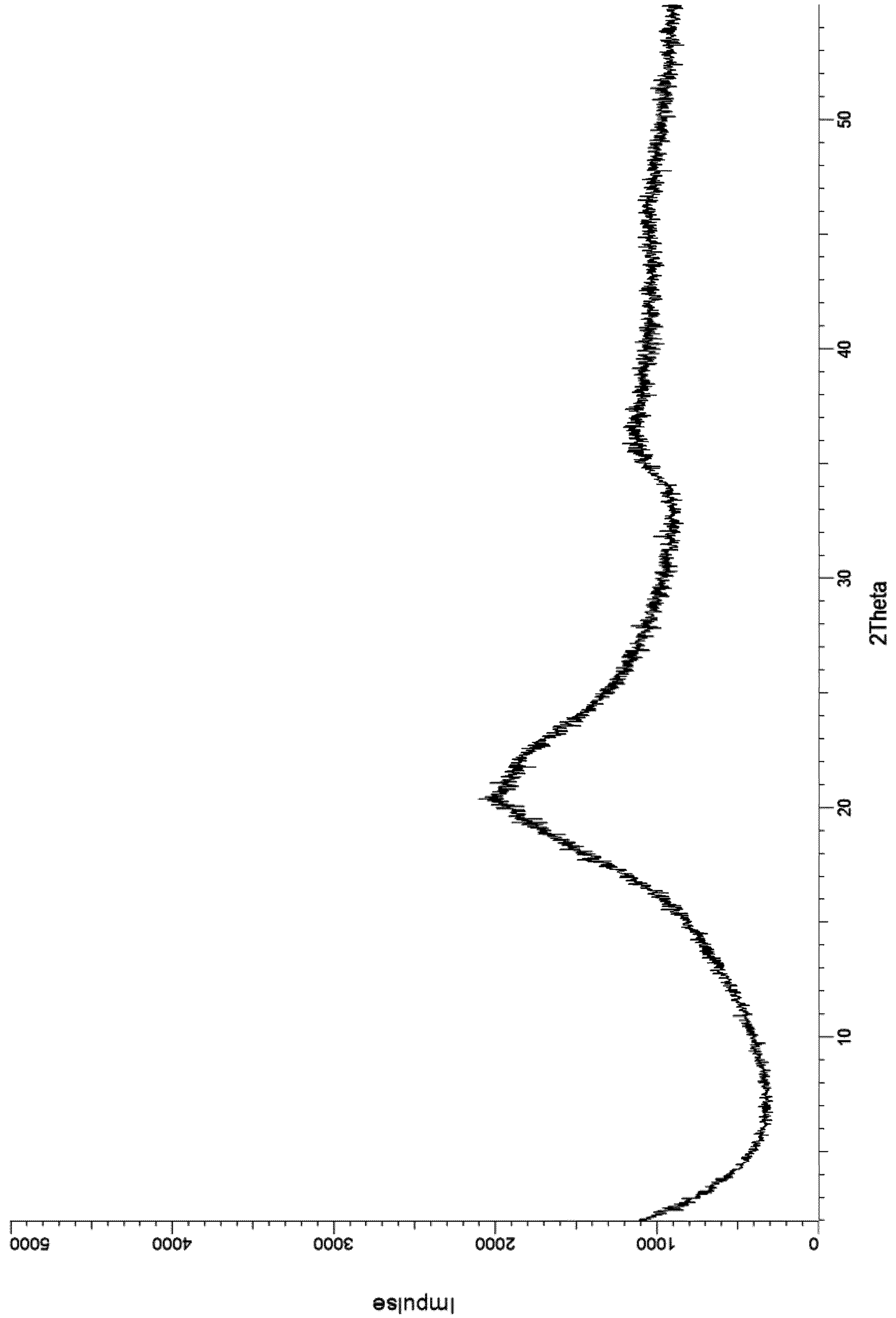


**Figure A-2: X-ray powder diffractogram of Example 1 (Cebranopadol in Glycofurol on Neusilin®)**

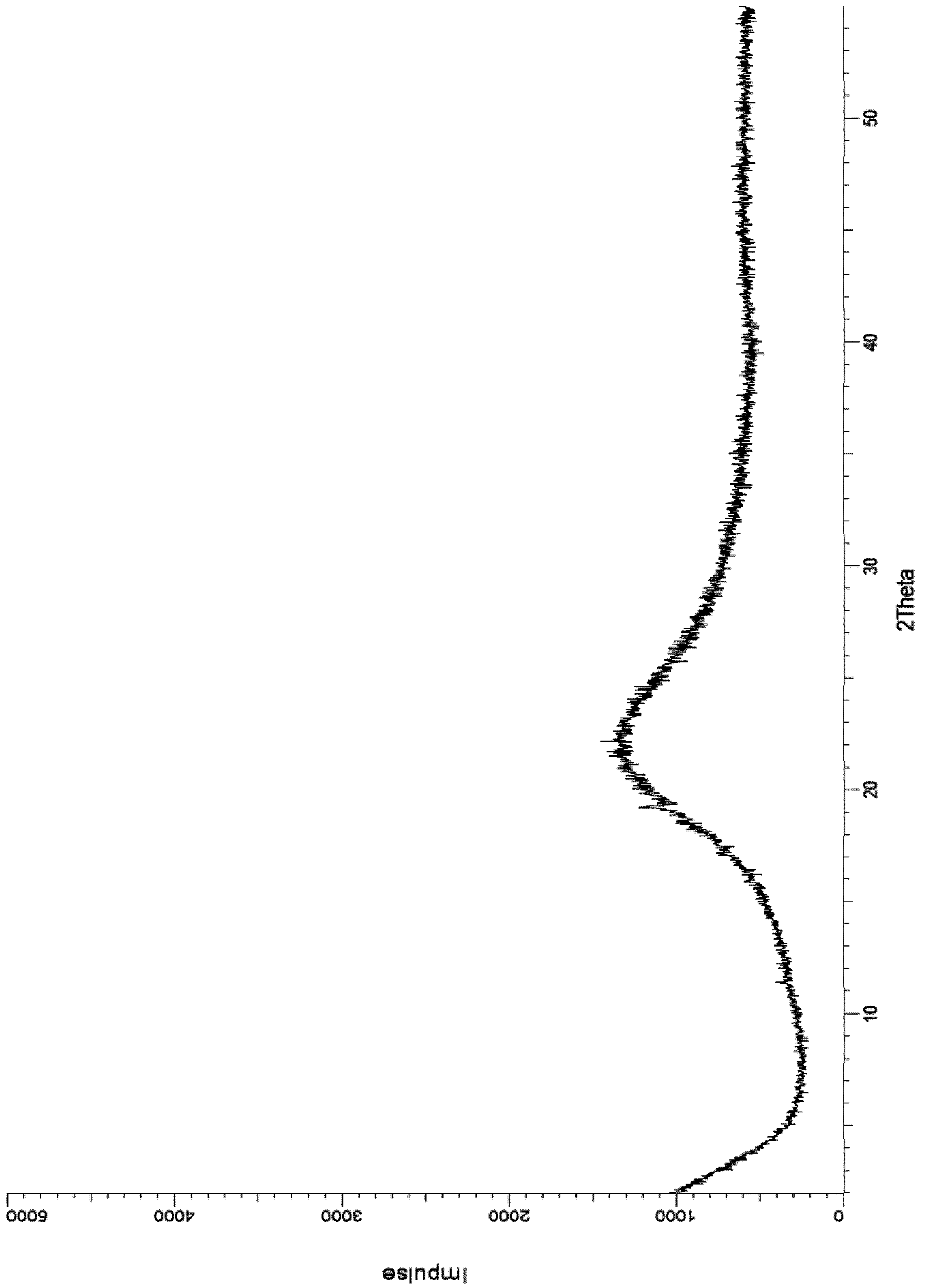


**Figure A-3: X-ray powder diffractogram of Example 2 (Cebranopadol in glycofurol on SBA-16)**

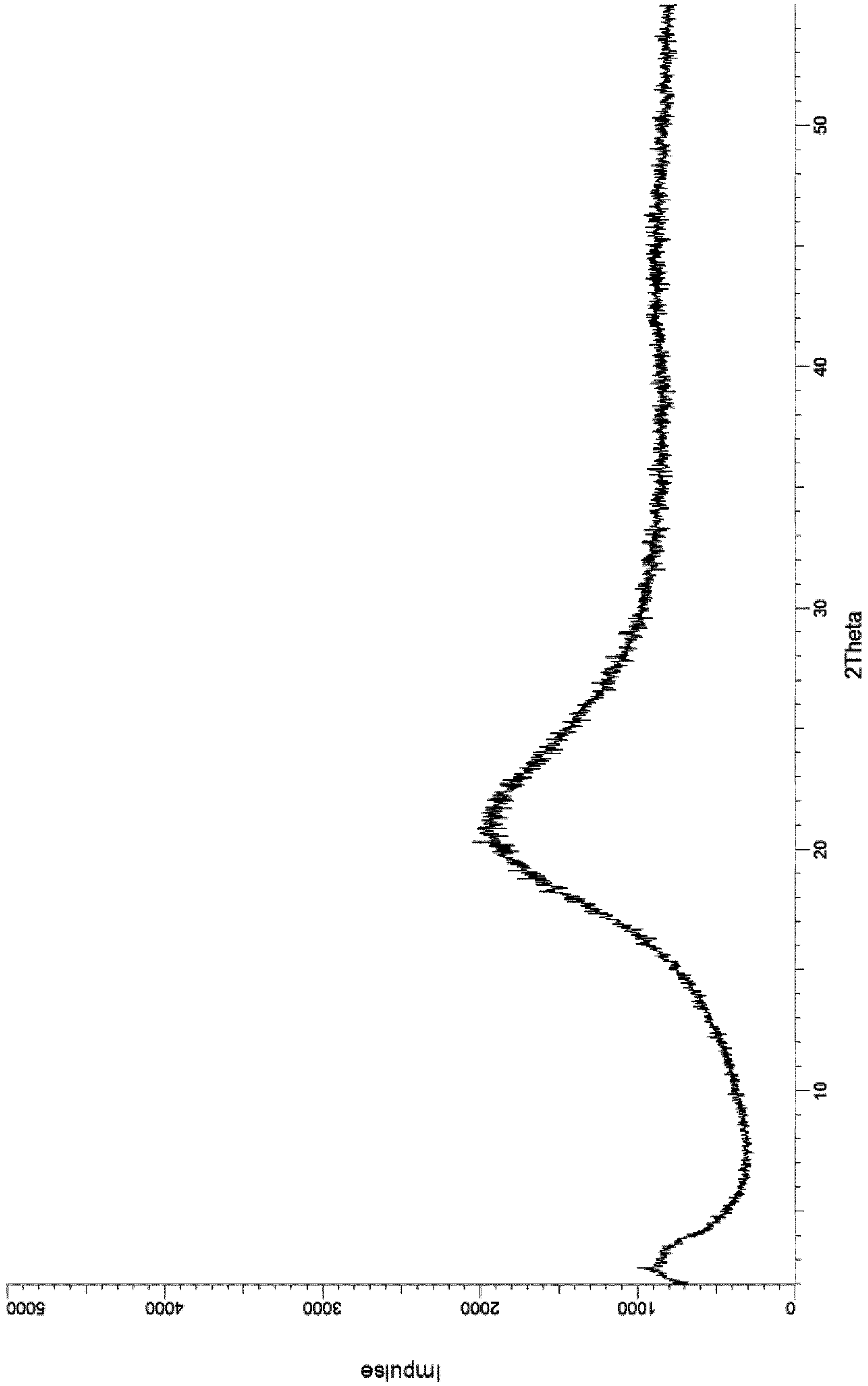


**Figure A-4: X-ray powder diffractogram of Example 3 (Cebranopadol in glycofurol on SBA-15).**

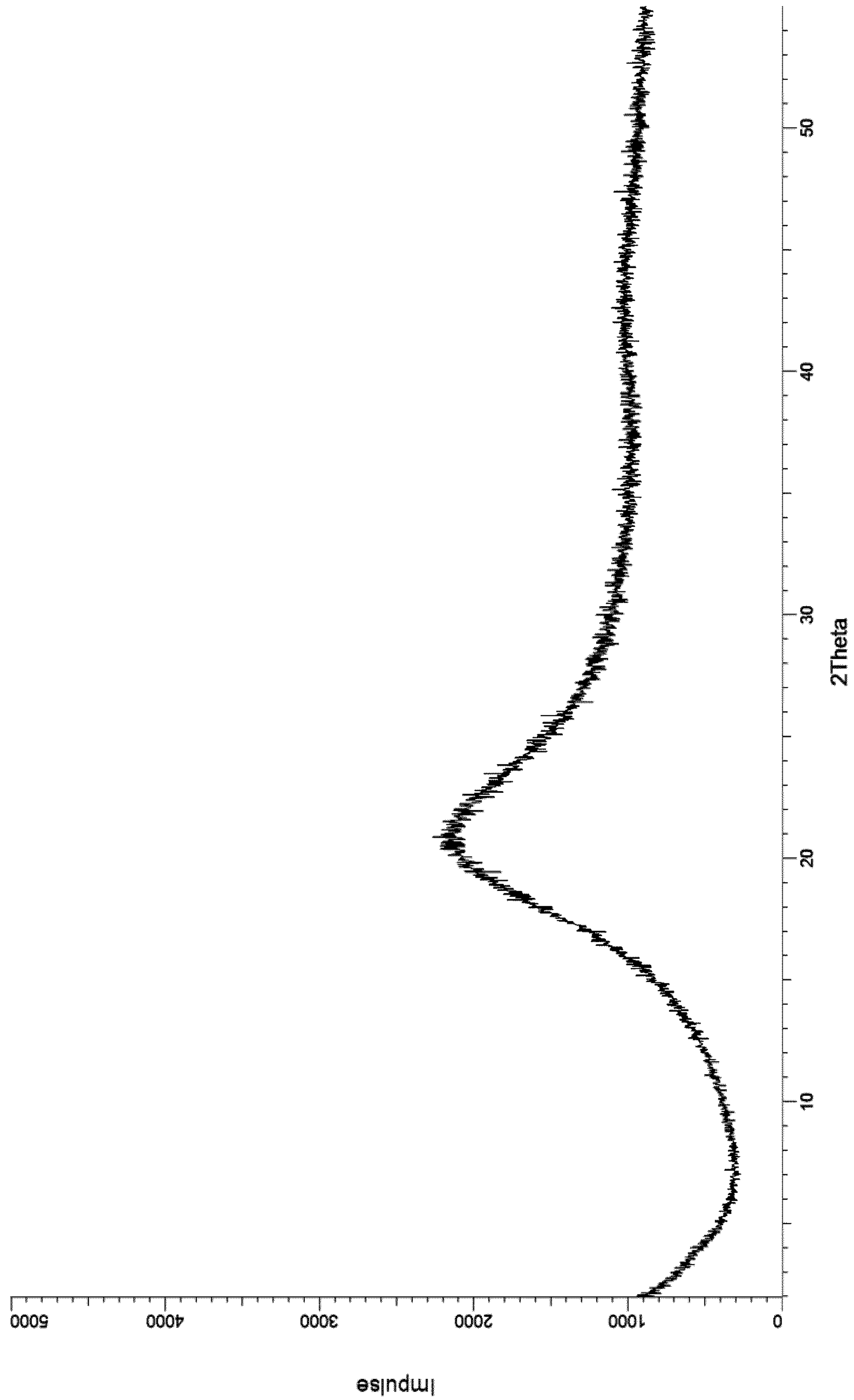


**Figure A-5: X-ray powder diffractogram of Example 4 (Cebranopadol in glycofurol on MSU-F)**

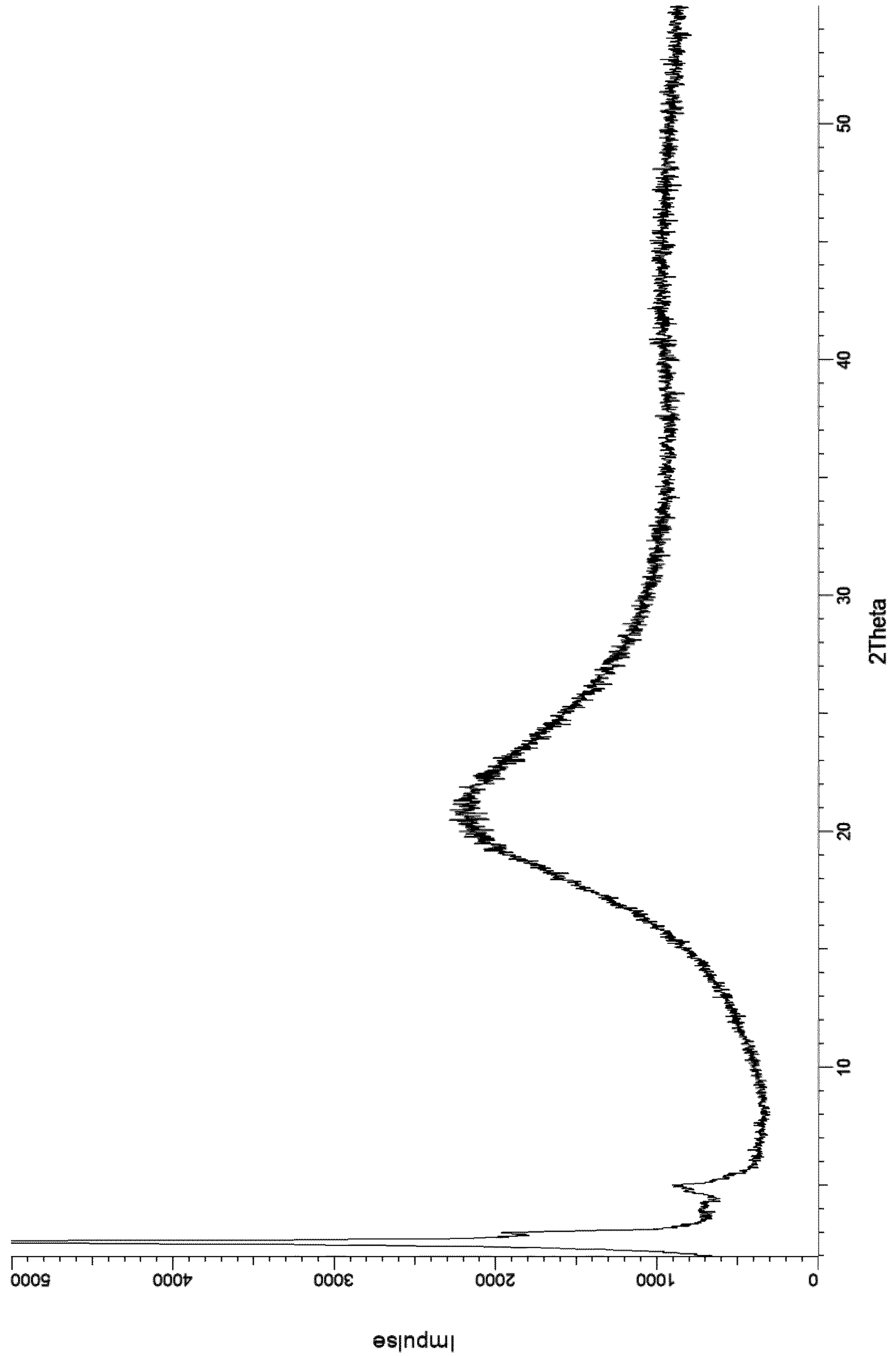


**Figure A-6: X-ray powder diffractogram of Example 5 (Cebranopadol in glycofurol on MCM-48)**

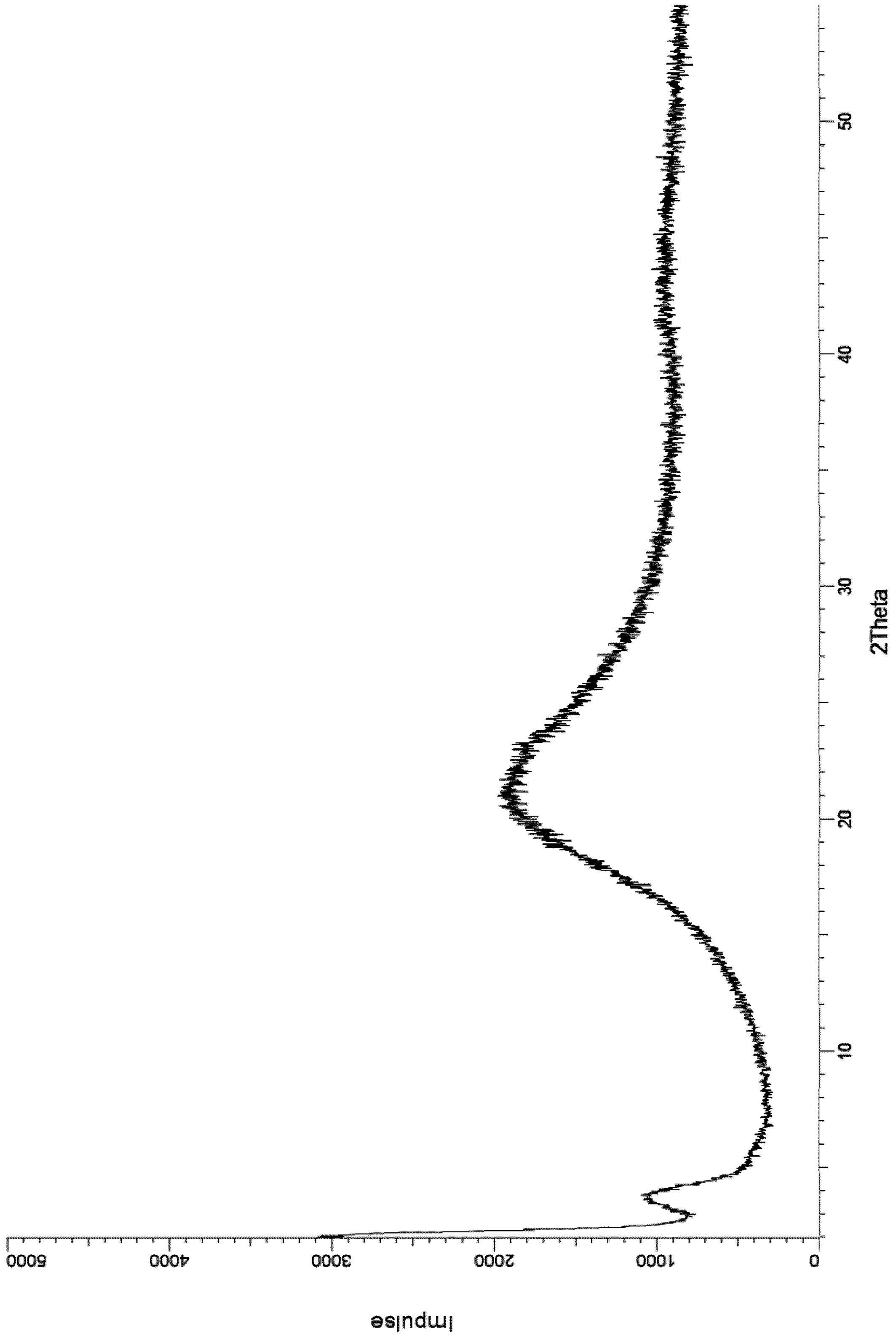


**Figure A-7: X-ray powder diffractogram of Example 6 (Cebranopadol in glycofurol on Al-MCM-41)**

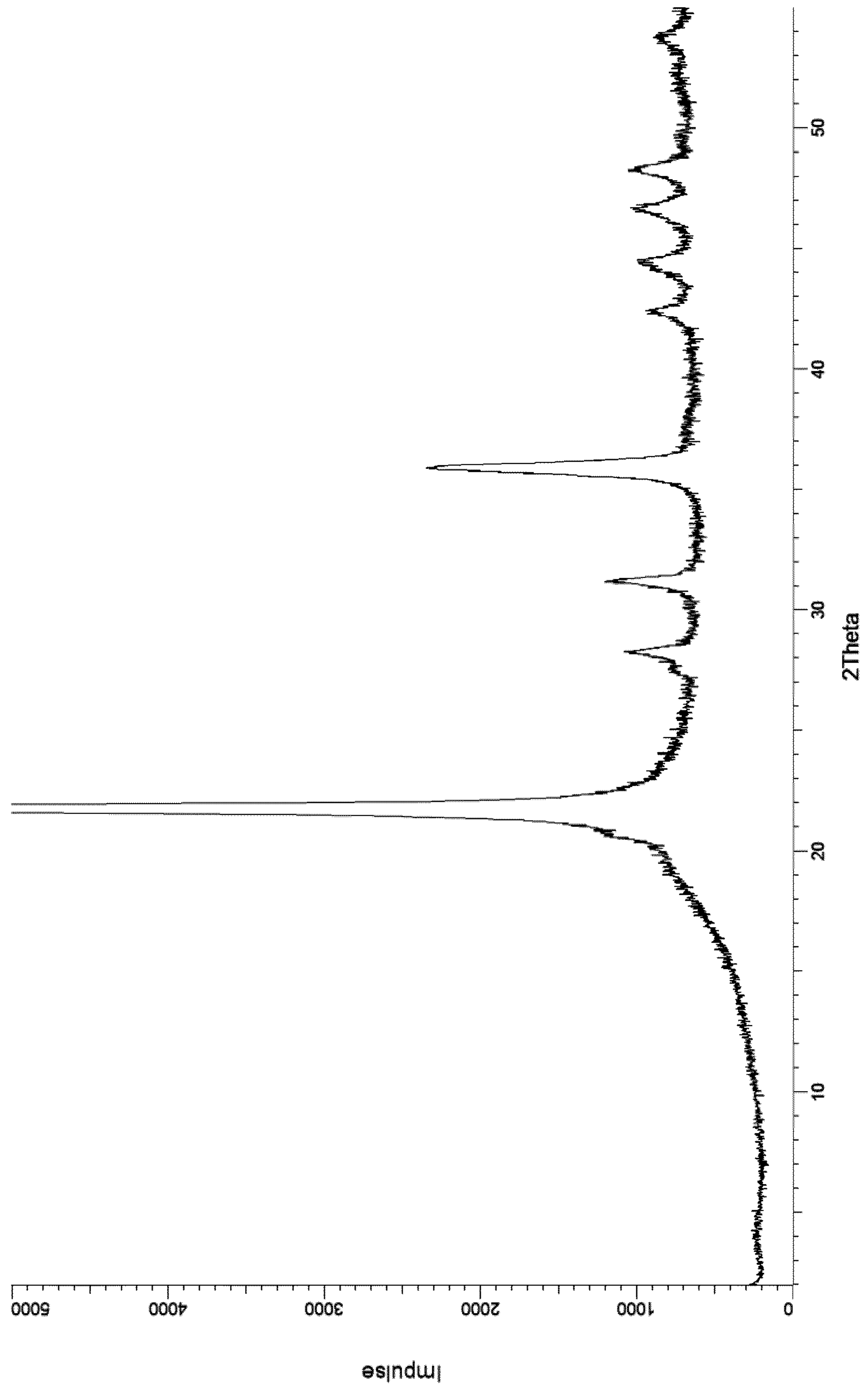


**Figure A-8: X-ray powder diffractogram of Example 7 (Cebranopadol in glycofurol on celite)**

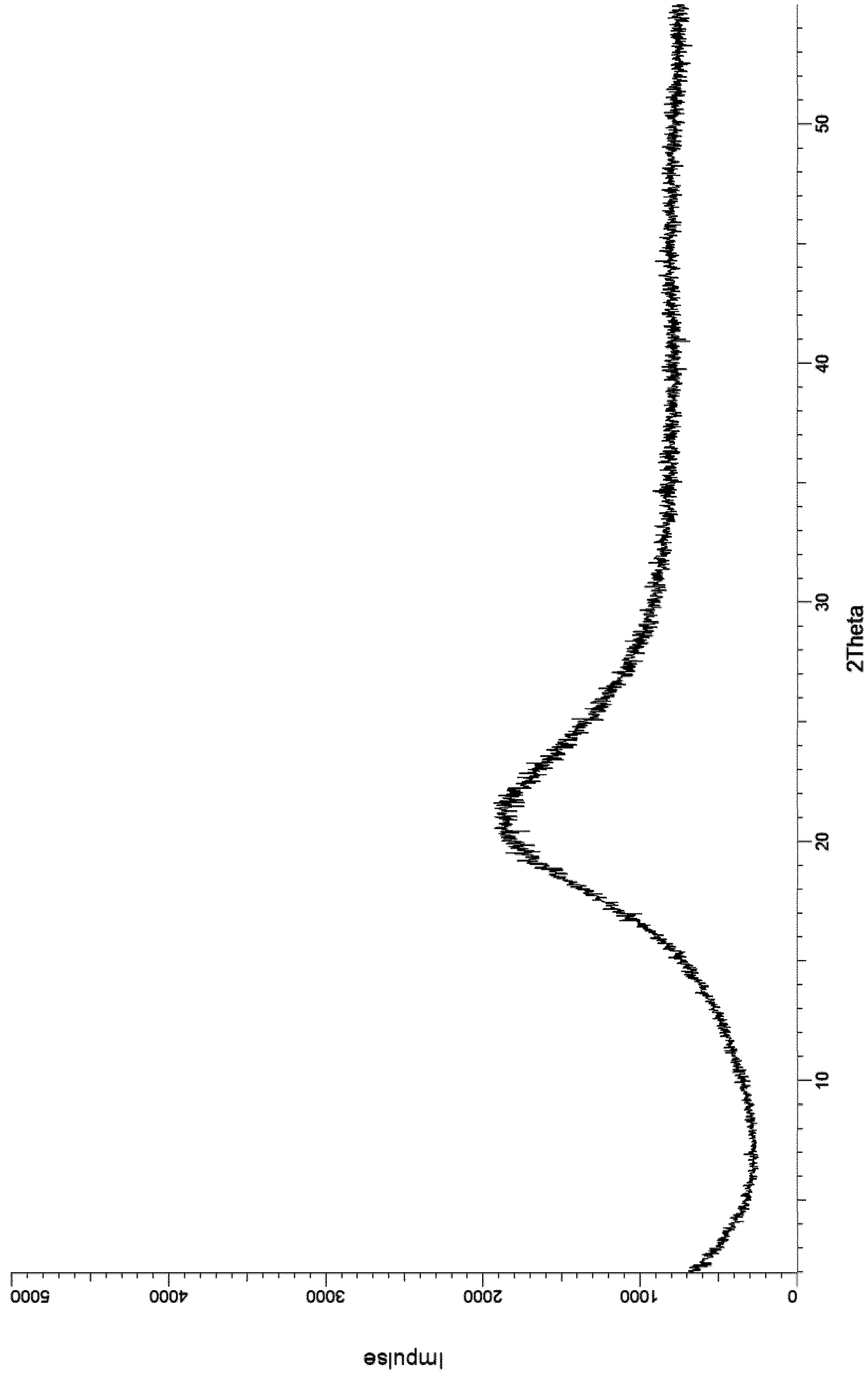


**Figure A-9: X-ray powder diffractogram of Example 8 (Cebranopadol in glycofurol on Aeroperl® 300)**

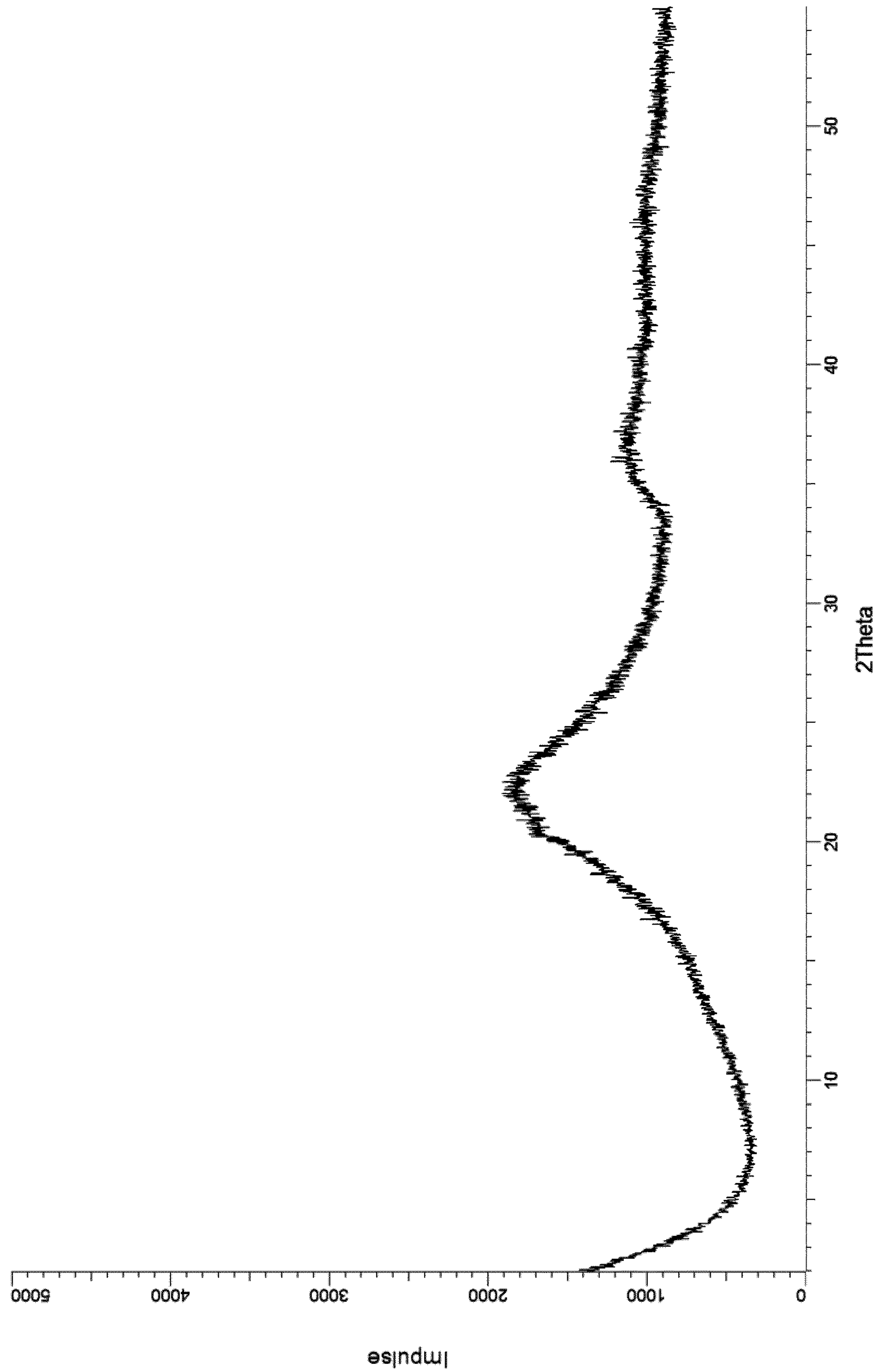


**Figure A-10: X-ray powder diffractogram of Example 9 (Cebranopadol in diethylene glycol monoethyl ether on Neusilin®)**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 1560835 B1
- WO 2013007361 A1
- WO 2012016703 A2
- WO 2012016703 A
- WO 2013007361 A


### Non-patent literature cited in the description


- **DUBERG, M. ; NYSTRÖM, C.** Studies on direct compression of tablets VI. Evaluation of methods for the estimation of particle fragmentation during compaction. *Acta Pharm. Suec.,* 1982, vol. 19, 421-436
- **HUMBERT-DROZ P. ; MORDIER D. ; DOELKER E.** Méthode rapide de determination du comportement a la compression pour des études de preformulation. *Pharm. Acta Helv.,* 1982, vol. 57, 136-143
- **YORK, P.** *Drug Dev. Ind. Pharm.,* 1992, vol. 18, 677
- Ph. Eur.